# EUROPEAN PATENT APPLICATION

(11) **EP 4 393 513 A1**
(43) Date of publication of application: **03.07.2024**
(21) Application number: 22860577.0
(22) Date of filing: 25.08.2022
(51) Int. Cl.: A61K 47/36, A61K 47/42, A61K 9/16, A61K 31/4439, A61P 1/00, A61P 29/00, A61P 1/04, A61P 37/08, A61P 1/14, A61P 31/04, A61P 7/04

(54) **DRY SUSPENSION PARTICLES FOR DRY SUSPENSION, PREPARATION METHOD THEREFOR AND USE THEREOF**

(30) Priority: 26.08.2021 CN 202110990993
(71) Applicant: Livzon Pharmaceutical Group Inc., Guangdong 519090 (CN)
(72) Inventor: LI, Pucheng, Zhuhai, Guangdong 519090 (CN); MO, Yating, Zhuhai, Guangdong 519090 (CN); ZHANG, Xiangna, Zhuhai, Guangdong 519090 (CN); HU, Siwen, Zhuhai, Guangdong 519090 (CN); HOU, Xuemei, Zhuhai, Guangdong 519090 (CN); JIANG, Xiaoman, Zhuhai, Guangdong 519090 (CN); LIN, Weishan, Zhuhai, Guangdong 519090 (CN); HAN, Zhihui, Zhuhai, Guangdong 519090 (CN); CUI, Yannan, Zhuhai, Guangdong 519090 (CN); CHENG, Caihua, Zhuhai, Guangdong 519090 (CN)
(74) Representative: Elzaburu S.L.P.
(86) International application number: PCT/CN2022/114705
(87) International publication number: WO 2023/025231

(57) **Abstract**

The present invention relates to dry suspension particles used for dry suspension, dry suspension containing the dry suspension particles, a preparation method therefor and a use thereof. The dry suspension particles comprise an anionic gel and a cationic polymer, wherein the weight ratio of the anionic gel to the cationic polymer is (0.5-50): 1.

## Description

### TECHNICAL FIELD

The present invention relates to dry suspension granules, suspension comprising the dry suspension granules, and a preparation method and use thereof.

### BACKGROUND

Proton Pump Inhibitors (PPIs) are medicament that can selectively inhibit the H⁺/K⁺-ATPase (also known as Proton Pump or Acid Pump) on the tubular membrane secreted by human gastric mucosa cells. Since the H⁺/K⁺-ATPase is the ultimate pathway to inhibit the secretion of gastric acid, it is possible to obviously reduce the secretion of gastric acid by inhibiting the H⁺/K⁺-ATPase. Therefore, the proton pump inhibitors are usually used to treat digestive tract diseases induced or caused by the effects of gastric acid (i.e. the acid-related diseases). These digestive tract diseases include gastric and duodenal ulcer, gastroesophageal reflux disease, surgical anastomotic ulcer, and Zollinger-Ellison syndrome. By the mechanism of action, the known PPIs may be classified into irreversible PPIs and reversible PPIs (RPPIs). Wherein, the irreversible PPIs are mainly benzimidazole derivatives, can pass through the parietal cell membrane quickly and accumulate in highly acidic secretory tubules. Then, the irreversible PPIs are protonated and transformed into sulfenamide compounds, the latter may be covalently bound to the sulfhydryl groups on cysteine residues in the α subunits of H⁺/K⁺-ATPase to form disulfide bonds, thereby irreversibly inactivating H⁺/K⁺-ATPase, and inhibiting its acid-secreting activity (Zhang Xuan, "Overview of Patent Technology for Proton Pump Inhibitors-Prazole Drugs"; Patent Literature Research in 2018 - Medical & Pharmaceutical, Intellectual Property Publishing House, Beijing, September. 2019: p554-567). So far, these drugs globally available in the market include: omeprazole, lansoprazole, pantoprozole, rabeprazole, esomeprazole, Ilaprazole, delansoprazole, etc.

Similar to other prazole drugs, Ilaprazole belongs to acid-unstable compounds. However, compared with other existing prazole drugs, Ilaprazole has a lower stability. The acid-unstable compounds refer to the substances that are unstable in acidic media but have better stability in neutral and alkaline media. The common feature of the compounds is that they can become biologically effective compounds after rapid degradation/transformation in acid media. The acid-unstable proton pump inhibitors are sensitive to degradation/transformation in acidic and neutral media. Therefore, the acid-unstable proton pump inhibitors shall be prevented from contacting with gastric acid when being administered orally in order not to affect the stability thereof. One of the solutions for this problem is to prepare the enteric pellet preparations by coating the oral preparations of these drugs with enteric materials.

The enteric pellets are usually not administered by direct administration routes, but further made into other forms of dosage such as tablets, capsules or dry suspensions, so as to facilitate the ministration. The dried suspension is a kind of powder or granule material made of insoluble solid drugs together with appropriate excipients, prior to use, the dry suspension can be dispersed into a suspension solution by shaking with water before use. In addition to water, other liquid dispersion media and additives suitable for oral administration may be added into the dry suspension to improve the taste and other properties. Suspension is a process in which the insoluble solid granules are dispersed almost uniformly in an appropriate liquid dispersion medium by a mechanical method. Due to a high dispersion degree, the solid granules in the formed suspension solution have a higher surface free energy, are more likely to conglomerate, so the suspension solution is a thermodynamically unstable system. However, because the solid granules in the suspension solution are larger than colloidal particles, they are prone to sedimentation by gravity, and therefore the suspension solution is also a dynamically unstable system.

Therefore, how to make improvements on the suspension, in particular, in the thermodynamic and kinetic properties of the dry suspensions comprising pellets when added into a dispersion medium (especially water) to prepare the suspensions, for example, the time how long to reach a stable viscosity level, and the time cost which the solid granules (such as pellets) are suspended stably, have become issues faced in this field.

### SUMMARY OF THE INVENTION

Surprisingly, the inventors discovered that, by using combinations of anionic gels and cationic polymer (e.g. combinations of chitosan and its derivatives) in the preparation of dry suspension granules, the prepared suspension may reach a stable viscosity level quickly, and the prepared suspension gel may allow pellets to suspend stably for a long period of time.

The purpose of the present invention is fulfilled with the dry suspension granules which comprises anionic gels and cationic polymer according to the present invention, in particular the composition of the dry suspension granules may allow the pellets to suspend stably in the suspension gel after forming a suspension solution /suspension gel. Therefore, through the dry suspension granules according to the present invention, it can provide a suspension capable of forming a stable suspension gel more quickly, and allow pellets in the suspension gel formed by the suspension to suspend stably for a longer period of time.

Therefore, in one aspect, the present invention provides a dry suspension granules comprising anionic gels and cationic polymer, wherein the weight ratio of the anionic gels to the cationic polymer is (0.5-50): 1.

According to an embodiment of the present invention, preferably, the weight ratio of the anionic gels to the cationic polymer is (0.8~20):1, more preferably (0.9~10):1, and most preferably (1-3): 1.

According to an embodiment of the present invention, the anionic gels is selected from one, two or more of arabic gum, gelatin, alginate (e.g. sodium alginate), pectin, xanthan gum, gellan gum, locust bean gum, guar gum, agar, carrageenan, tamarind gum, konjac gum, cassia bean gum, tragacanth gum, and karaya gum.

According to an embodiment of the present invention, the cationic polymer is selected from chitosan or a derivative thereof.

According to an embodiment of the present invention, the weight percentage of the cationic polymer in dry suspension granules is 0.5-5%, preferably 0.6-4%, more preferably 0.8-3.5%, and most preferably 0.9-3.0%.

According to an embodiment of the present invention, the dry suspension granules further comprise one, two or more of adhesives, disintegrants, diluents, and pH regulators.

According to an embodiment of the present invention, the adhesives are selected from one, two or more of polyvinyl pyrrolidone, hydroxypropyl methyl cellulose, hydroxypropyl cellulose, methyl cellulose, polyvinyl alcohol, and starch.

According to an embodiment of the present invention, the disintegrants are selected from one, two, or more of sodium carboxymethyl cellulose, calcium carboxymethyl cellulose, crosslinked sodium carboxymethyl cellulose, crosslinked sodium carboxymethyl starch, low-substituted hydroxypropyl cellulose, crosslinked polyvinyl pyrrolidone, microcrystalline cellulose, and pre-gelatinized starch.

According to an embodiment of the present invention, the diluents are selected from one, two or more of xylitol, mannitol, sucrose, glucose, sorbitol, maltitol, and fructose.

According to an embodiment of the present invention, the pH regulators are selected from organic or inorganic acids, preferably one, two or more of tartaric acid, citric acid, oxalic acid, succinic acid, fumaric acid, ascorbic acid, malic acid, glutamic acid, and caffeic acid.

According to an embodiment of the present invention, the pH value of suspension gel formed by the dry suspension granules in aqueous media ranges from 2.5 to 7.0, and preferably ranges from 3.0 to 5.0.

According to an embodiment of the present invention, when preparing a suspension gel, the amount of an aqueous dispersion medium added into the dry suspension granules according to the present invention is 2 to 50 times of the weight of the dry suspension granules.

In another aspect, the present invention provides a method for preparing the dry suspension granules according to the present invention, comprising the following steps:
(1) mixing anionic gels and cationic polymer, and optionally other excipients;
(2) preparing adhesive solutions separately;
(3) adding the adhesive solutions obtained in step (2) into the mixture obtained in step (1), to prepare a wetting mixture;
(4) granulating the wetting mixture obtained in step (3), to obtain the dry suspension granules according to the present invention.

Preferably, the excipients comprise diluents, pH regulators, disintegrants, etc.

Preferably, water and/or ethanol are used as solvent in step (2).

In another aspect, the present invention provides a method for preparing the dry suspension granules according to the present invention, comprising the following steps:
(1) mixing anionic gels and cationic polymer, and optionally other excipients;
(2) providing water and/or ethanol as wetting agents;
(3) adding the wetting agents provided in step (2) into the mixture obtained in step (1), to prepare a wetting mixture;
(4) granulating the wetting mixture obtained in step (3), to obtain the dry suspension granules according to the present invention.

Preferably, the other excipients comprise diluents, pH regulators, disintegrants, and/or adhesives, etc.

Preferably, in the method for preparing dry suspension granules according to the present invention, further comprising a step (5) after step (4); step (5): drying the dry suspension granules.

Preferably, in the method for preparing the dry suspension granules according to the present invention, further comprising a step (6) after step (5); step (6): granulating the dry suspension granules in the form of pellet.

In another aspect, the present invention also provides a pharmaceutical composition, in particular a dry suspension comprising dry suspension granules according to the present invention.

According to an embodiment of the present invention, the present invention also provides a pharmaceutical composition, in particular a dry suspension comprising the dry suspension granules and enteric pellets (especially the enteric pellets comprising Ilaprazole) according to the present invention.

According to an embodiment of the present invention, the mass ratio of the dosage of Ilaprazole in the enteric pellets to the dosage of the dry suspension granules in the pharmaceutical composition is 1:200~1000.

According to an embodiment of the present invention, after adding a dispersing medium to the pharmaceutical composition according to the present invention, the time taken for the obtained suspension gel to reach 75% of the maximum viscosity is less than 8 min, preferably 7 min.

According to an embodiment of the present invention, after adding a dispersing medium to the pharmaceutical composition according to the present invention, the time taken for the obtained suspension gel to reach 90% of the maximum viscosity is less than 12 min, preferably less than 11 min.

According to an embodiment of the present invention, preferably, the time for forming a suspension gel is less than 120 s, preferably less than 90 s, more preferably less than 70 s, and most preferably less than 50 s, wherein the suspension gel is formed after adding an aqueous medium into the pharmaceutical composition according to the present invention.

According to an embodiment of the present invention, preferably, the release rate of Ilaprazole within 1h not exceed 10% after forming a solution having a pH 1.2 by adding the pharmaceutical composition, into a dispersion medium according to the present invention.

According to an embodiment of the present invention, preferably, the release rate of Ilaprazole within 45 min is not less than 70% after forming a solution having a pH 6.8 by adding the pharmaceutical composition into a dispersion medium according to the present invention.

In the ongoing animal experiments and studies, the inventors discovered that, the preparations containing enteric pellets according to the present invention, in particular the suspension, shown beneficial effects in aspects of treating and/or preventing gastrointestinal diseases, wherein the gastrointestinal diseases mainly comprise heartburn, inflammatory bowel disease, Crohn's disease, irritable bowel syndrome, ulcerative colitis, peptic ulcer, stress ulcer, bleeding peptic ulcer, duodenal ulcer and recurrent duodenal ulcer, gastric ulcer associated with NSAID, adult active benign gastric ulcer, infective enteritis, colitis, hyperacidity, dyspepsia, gastroparesis, Zollinger-Ellison syndrome, gastroesophageal reflux disease (GERD), diseases related to helicobacter pylori or eradication of helicobacter pylori, erosive esophagitis at all levels, short bowel syndrome, or any combination of the above diseases.

Therefore, according to one aspect of the present invention, the present invention provides a method for treating and/or preventing gastrointestinal diseases. The method comprises a step of administering an effective dose of the suspension of the present invention to patients in need of this treatment and/or prevention. Wherein, the gastrointestinal diseases that can be treated and/or prevented by this method include but are not limited to heartburn, inflammatory bowel disease, Crohn's disease, irritable bowel syndrome, ulcerative colitis, peptic ulcer, stress ulcer, bleeding peptic ulcer, duodenal ulcer and recurrent duodenal ulcer, gastric ulcer associated with NSAID, adult active benign gastric ulcer, infective enteritis, colitis, hyperacidity, dyspepsia, gastroparesis, Zollinger-Ellison syndrome, gastroesophageal reflux disease (GERD), diseases related to helicobacter pylori or eradication of helicobacter pylori, erosive esophagitis at all levels, short bowel syndrome, or any combination of the above diseases.

Correspondingly, according to another aspect of the present invention, the present invention provides use of the dry suspension of the present invention in the preparation of medicament for treating and/or preventing gastrointestinal diseases; the gastrointestinal diseases include, but are not limited to: heartburn, inflammatory bowel disease, Crohn's disease, irritable bowel syndrome, ulcerative colitis, peptic ulcer, stress ulcer, bleeding peptic ulcer, duodenal ulcer and recurrent duodenal ulcer, gastric ulcer associated with NSAID, adult active benign gastric ulcer, infective enteritis, colitis, hyperacidity, dyspepsia, gastroparesis, Zollinger-Ellison syndrome, gastroesophageal reflux disease (GERD), diseases related to helicobacter pylori or eradication of helicobacter pylori, erosive esophagitis at all levels, short bowel syndrome, or any combination of the above diseases.

Further, when the dry suspension of the present invention is used in preparation of medicament for treating and/or preventing gastrointestinal diseases, the gastrointestinal diseases include, but are not limited to, duodenal ulcer and recurrent ulcer, gastric ulcer, gastroesophageal reflux disease (GERD), diseases related to helicobacter pylori; or, the drugs may be used to eradicate helicobacter pylori, prevent peptic ulcer diseases caused by non-steroidal anti-inflammatory drugs, and prevent gastrointestinal bleeding and related ulcers caused by anti-platelet aggregation drugs (including, but not limited to clopidogrel, prasugrel, and ticagrelor).

### BRIEF DESCRIPTION OF THE DRAWINGS

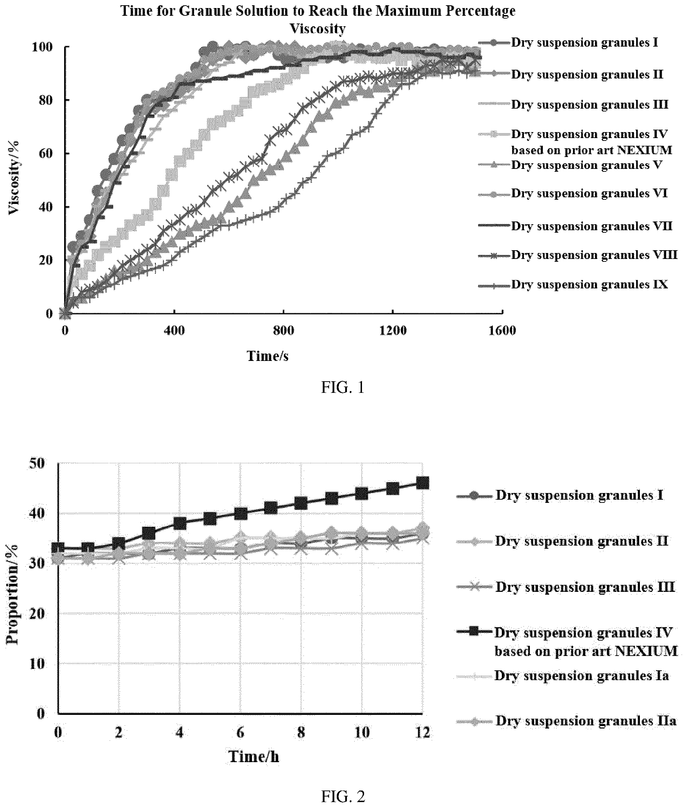

Figure 1 shows the time for the dry suspension granules I-III of the present invention, dry suspension granules IV in prior art, and dry suspension granules V in comparative example to reach the maximum percentage of viscosity after adding water as the dispersion medium.
Figure 2 shows the pellet suspension-sedimentation time for the dry suspensions having a volume of 0-5mL measured in a 15mL volumetric cylinder, the dry suspensions were prepared by adding different pellets into the suspension granules I-III of the present invention and the dry suspension granules IV in prior art.

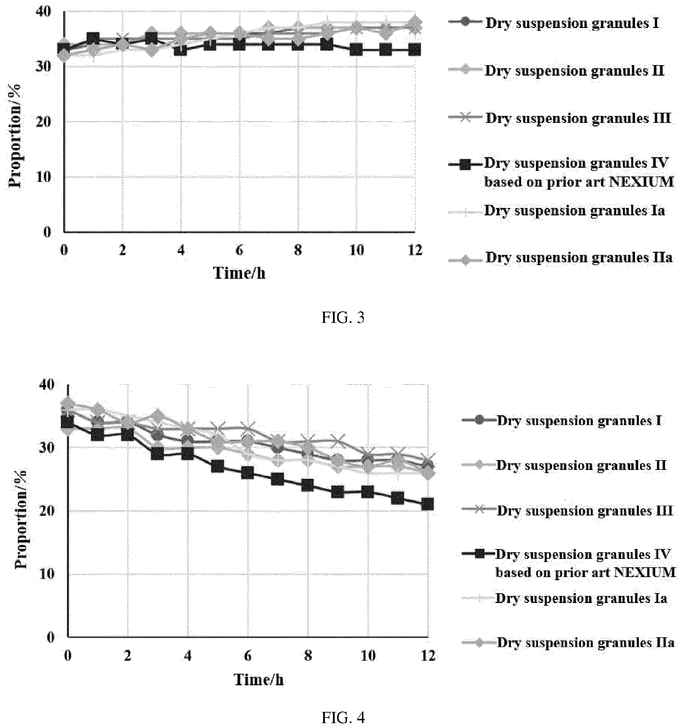

Figure 3 shows the pellet suspension-sedimentation time for the dry suspensions having a volume of. 5-10 mL measured in a 15mL volumetric cylinder, the dry suspensions were prepared by adding different pellets into the dry suspension granules I-III of the present invention and the dry suspension granules IV in prior art.
Figure 4 shows the pellet suspension-sedimentation time for the dry suspensions having a volume of 10-15mL measured in a 15mL volumetric cylinder, the dry suspensions were prepared by adding different pellets into the dry suspension granules I-III of the present invention and the dry suspension granules IV in prior art.

### Beneficial Effects of the Invention

Compared with prior art, the dry suspension granules and the dry suspension comprising the dry suspension granules of the present invention can disperse rapidly in a aqueous medium to form a stable suspension gel and obtain a uniform and stable dispersion system comprising suspended pellets in the presence of pellets simultaneously; the obtained solution/gel can reach a stable viscosity in a shorter period of time than that of dry suspension granules and dry suspensions in prior art, and maintain the pellets in a stable suspending state for a longer period of time.

For acid-unstable proton pump inhibitors, especially Ilaprazole, it can additionally enhance the dissolution rate through the suspension solution obtained from the dry suspension according to the present invention.

### DETAILED DESCRIPTION

In a preferable embodiment of the present invention, it provides a dry suspension comprises enteric pellets, preferably Ilaprazole enteric pellets, and dry suspension granules. The dry suspension may include, but not limited to, the following components in parts by weight:

| | |
|---|---|
| Enteric pellets | 1∼30; |
| Gel | 0.1∼15; |
| Adhesive | 0.1∼20; |
| Disintegrant | 0.1∼15; |
| Diluent | 1∼90; |
| pH regulator | 0.02∼6; |
| Cationic polymer | 0.1∼15. |

In terms of the enteric pellets, the pellets in prior art may be used or the enteric pellets in the following embodiments may be used preferably according to the various aspects of the present invention.

According to the present invention, in the first embodiment of the enteric pellets, the enteric pellets include a pellet core, a first isolating layer, a second isolating layer, and an enteric layer from inside to outside; wherein the pellet core contains Ilaprazole and/or pharmaceutically acceptable salts thereof, and a first excipient, which is characterized by the fact that, the first isolating layer comprises a water-insoluble alkaline compound, and the first excipient is a water-insoluble alkaline compound; the weight ratio of the first excipient to Ilaprazole and/or pharmaceutically acceptable salts thereof is 0.2~5:1.

According to the present invention, in the second embodiment of the enteric pellets, the enteric pellets include the pellet core, the first isolating layer, the second isolating layer, and the enteric layer from inside to outside; wherein, the pellet core contains Ilaprazole and/or pharmaceutically acceptable salts thereof and the first excipient; the enteric pellets are characterized by the fact that it also comprises a water-insoluble alkaline compound in the first isolating layer and the first excipient is a water-insoluble alkaline compound; the a water-insoluble alkaline compound in the first isolating layer may be the same as or different from the water-insoluble alkaline compound used as the first excipient.

According to the present invention, in the third embodiment of the enteric pellets, the enteric pellets include the pellet core, the first isolating layer, the second isolating layer, and the enteric layer from inside to outside; wherein, the pellet core contains Ilaprazole and/or pharmaceutically acceptable salts thereof and the first excipient; the enteric pellets are characterized by the fact that the particle size (D90) of Ilaprazole and/or pharmaceutically acceptable salts thereof is less than or equal to 100µm, and it also comprises no alkaline substance in the second isolating layer.

Preferably, it also comprises a protective layer on the outside of the enteric layer of the enteric pellets according to the present invention.

Preferably, there exists no other layer between the pellet core and the first isolating layer of the enteric pellets according to the present invention.

Preferably, there exists no other layer between the second isolating layer and the enteric layer of the enteric pellets according to the present invention.

Preferably, there exists no other layers between the first isolating and the second isolating layer of the enteric pellets according to the present invention.

Preferably, the first excipient in the enteric pellets of the present invention is an alkaline compound, preferably a water-insoluble alkaline compound, and more preferably magnesium hydroxide, aluminum hydroxide, magnesium oxide, magnesium carbonate, calcium carbonate, and calcium hydroxide.

Preferably, the pharmaceutically acceptable salts thereof may be, for example, Ilaprazole sodium, Ilaprazole magnesium, Ilaprazole zinc, Ilaprazole potassium, Ilaprazole lithium, or Ilaprazole calcium, and so on. For this purpose, the technical personnel in the field may select the appropriate salt according to their needs.

Preferably, the pellet core of the enteric pellets according to the present invention also contains surfactant. Preferably, the surfactant is Tween 80 or sodium dodecyl sulfate.

Preferably, the weight ratio of the water-insoluble alkaline compound in the first isolating layer to Ilaprazole and/or pharmaceutically acceptable salts thereof is 0.2-5:1, preferably 0.25∼4:1, more preferably 0.3∼3:1, particularly preferably 0.5∼2:1, and most preferably 0.8∼1.2:1, e.g. 1: 1.

Preferably, in the second isolating layer, it also comprises the water insoluble inert substance that can prevent the adhesion of pellets, and the dosage ratio of the water insoluble inert substance to the adhesive by weight ranges from 1-8 : 1.5∼10, or 1~10 : 1~20, or 4~26 : 7∼44.

Preferably, the particle size (D90) of Ilaprazole and/or pharmaceutically acceptable salts thereof may be selected from the range between any two of the following endpoints: 0µm (excluding the value of this point when constituting the range) 10µm, 20µm, 30µm, 40µm, 50µm, 60µm, 70µm, 80µm, 90µm, and 100µm.

### Pellet core of Enteric Pellets:

The pellet core (or known as drug-containing pellet) of the enteric pellets according to the present invention may be a fully active pellet core or a blank pellet core coated with a drug loading layer. The term "fully active pellet core" referred to herein means the pellet core comprising Ilaprazole and/or pharmaceutically acceptable salts thereof and the first excipient, as well as one or more other pharmaceutical excipients; wherein, Ilaprazole and/or pharmaceutically acceptable salts thereof serving as the active ingredients are dispersed in other ingredients (the first excipient and one or more other pharmaceutical excipients, etc.), but do not form additional layers independently or together with any other ingredients; in the blank pellet core coated with a drug loading layer, the drug loading layer contains Ilaprazole and/or pharmaceutically acceptable salts thereof and the first excipient as well as optional excipient.

If Ilaprazole and/or pharmaceutically acceptable salts thereof in the pellet core cannot achieve sufficient storage stability only with the action of the water-insoluble alkaline compound contained in the first isolating layer, its storage stability may be improved by adding more quantity of the first excipient into the pellet core.

The first excipient in the pellet core may be a conventional excipient in prior art and used to improve the stability of acid-unstable compounds. Preferably, the first excipient is an alkaline compound, including a water-insoluble alkaline compound and a water-soluble alkaline compound. Preferably according to the present invention, a water-insoluble alkaline compound is used as the first excipient in the pellet core; more preferably, the water-insoluble alkaline compound contained in the pellet core is the same as the water-insoluble alkaline compound contained in the first isolating layer, so that it is possible to further enhance the buffering effect of the isolating layer on pH value. In the embodiments of the present invention, the water-insoluble alkaline compound may be selected from, but not limited to, one or more of magnesium hydroxide, aluminum hydroxide, magnesium oxide, magnesium carbonate, calcium carbonate, and calcium hydroxide. Preferably, the weight ratio of the alkaline compound to Ilaprazole and/or pharmaceutically acceptable salts thereof is 0.2-5:1, preferably 0.25-4:1, more preferably 0.3-3:1, particularly preferably 0.5-2:1, and most preferably 0.8∼1.2:1, e.g. 1:1.

According to the present invention, surfactant may also be contained in the pellet core. It is proved by the embodiments according to the present invention that the surfactant can increase the dissolution rate of Ilaprazole and/or pharmaceutically acceptable salts thereof in the enteric pellets and their preparations, so as to improve their bioavailability effectively. In the embodiments according to the present invention, the surfactant contained in the pellet core may be selected from non-ionic surfactant, anionic surfactant, and amphoteric surfactant. Preferably, the non-ionic surfactant may be selected from polyethylene glycol, polyol (e.g. Tween 80), etc. The anionic surfactant may be selected from higher fatty acid salts, sulfate ester salts, and sulfonates such as sodium dodecyl sulfate; the amphoteric surfactant may be selected from phosphorus esters.

According to the present invention, the particle size of Ilaprazole and/or pharmaceutically acceptable salts thereof may affect the dissolution rate and/or drug loading capacity of the enteric pellets. In a preferable embodiment of the present invention, the particle size (D90) of Ilaprazole and/or pharmaceutically acceptable salts thereof may be less than or equal to 100µm; in this case, enteric pellets have good dissolution rate, and the bioavailability of enteric pellet preparations made of enteric pellets may be improved. More preferably, the particle size (D90) of Ilaprazole and/or pharmaceutically acceptable salts thereof may be selected from the range between any two of the following endpoints: 10µm, 20µm, 30µm, 40µm, 50µm, 60µm, 70µm, 80µm, 90µm, and 100µm; in particular when D90 is less than or equal to 50µm, it is possible to achieve the improved drug loading capacity.

According to an embodiment of the present invention, the abovementioned blank pellet core is the blank pellet core conventionally used in prior art. In the embodiments according to the present invention, the blank pellet core may be selected from, but is not limited to, the microcrystalline cellulose pellet core, the sucrose pellet core, or the mannitol pellet core with the particle size of 50~500µm, preferably 100~400µm, more preferably 250~350µm, and most preferably about 300µm.

According to an embodiment of the present invention, the abovementioned drug loading layer may also contain adhesive. The adhesive may be selected from, but is not limited to, one or more of hydroxypropyl cellulose, hydroxypropyl methyl cellulose, hydroxymethyl cellulose, hydroxyethyl cellulose, carboxymethyl cellulose, methyl cellulose, polyvinyl pyrrolidone, polyvinyl alcohol, polyethylene glycol, syrup, and starch. In the embodiments according to the present invention, the adhesive may be one or more of hydroxypropyl cellulose-SSL (e.g. commercially available series from Nisso), hydroxypropyl methyl cellulose E5, polyvinyl pyrrolidone K30, polyvinyl alcohol, methyl cellulose, and polyethylene glycol.

### Isolating Layers of Enteric Pellets:

The basic function of the isolating layers of enteric pellets is to isolate the pellet core in an alkaline environment from the enteric layer comprising free carboxyl group, in order to prevent degradation or discoloration of Ilaprazole and/or pharmaceutically acceptable salts thereof during the process of coating or storage. In the research on Ilaprazole enteric pellets and their preparations, the inventors discovered that the water insoluble inert substance (e.g. talcum powder, silicon dioxide, titanium dioxide, magnesium stearate) and/or the water soluble alkaline compounds commonly used in conventional preparation method (e.g. when preparing the isolating layer closely adjacent to the pellet core) in prior art to prevent adhesion of pellets, as well as the alkaline compounds commonly used in preparation of the isolating layer closely adjacent to the enteric layer, can reduce the stability and/or acid resistance of the enteric pellets and their preparations; in particular, for the acid-unstable compounds with relatively low stability such as Ilaprazole, the enteric pellets and their preparations cannot meet the requirements for stability and acid resistance simultaneously as a result. The term "closely adjacent" referred to herein means that it also comprises no additional layers between the pellet core of enteric pellets and its coating or cladding layers, or between any two layers.

As one reason causing this technical problem, it is proved by experiments and believed by the inventors that (but not limited to), the compatibility between the water insoluble inert substance contained in the isolating layer (corresponding to the first isolating layer according to the present invention) closely adjacent to the pellet core to prevent adhesion of pellets and the acid-unstable compounds contained in the pellet core differs due to the different stability of the acid-unstable compounds; that is to say, in the enteric pellets and their preparations prepared according to prior art, if the stability of the acid-unstable compounds (e.g. Ilaprazole) contained in the pellet core is low, the compatibility between the water insoluble inert substance (e.g. talcum powder) contained in the isolating layer closely adjacent to the pellet core to prevent adhesion of pellets and the acid-unstable compounds will also be reduced. Therefore, even under the protection of alkaline compounds contained as stabilizers in the pellet core and/or the isolating layer, it also comprises still a significant increase in the relevant substances (i.e. impurities) in the accelerated test results, thus reducing the stability of enteric pellet preparations. This will limit, to some extent, the range of the acid-unstable compounds applicable to the enteric pellets prepared according to prior art and their preparations, that is to say, the prescriptions or the components of enteric pellets prepared according to prior art and their preparations cannot be applied properly to Ilaprazole and/or pharmaceutically acceptable salts thereof with relatively low stability. In addition, if the isolating layer (corresponding to the first isolating layer according to the present invention) closely adjacent to the pellet core contains water-soluble alkaline compound, the isolating layer of the enteric pellets will absorb free water and cause dissolution of the water-soluble alkaline compound in the condition of high temperature and high humidity for a long time, so that the alkalinity of the isolating layer closely adjacent to the enteric layer will increase and the isolating layer becomes alkaline; if water infiltrates into the enteric layer with the acidic medium, the enteric layer will dissolve in advance, resulting in the decrease in acid resistance of enteric pellets and their preparations. The principle that the enteric layer will dissolve in advance because the isolating layer closely adjacent to the enteric layer becomes alkaline is also applicable to the situation that the isolating layer (corresponding to the second isolating layer according to the present invention) closely adjacent to the enteric layer contains alkaline compounds.

Therefore, according to the present invention, the enteric pellets include at least two isolating layers comprising inert substance, i.e. the first isolating layer closely adjacent to the pellet core, and the second isolating layer which is far away from the pellet core compared with the first isolating layer or is closely adjacent to the enteric layer. Wherein, the first isolating layer contains a water-insoluble alkaline compounds, but does not contain water-soluble alkaline compounds and water insoluble inert substance that can prevent adhesion of pellets, and the second isolating layer does not contain alkaline compounds. If the enteric pellets include three or more isolating layers, the other isolating layers between the first and the second isolating layers may be an isolating layer conforming to the definition of the first or the second isolating layer according to the present invention, or an isolating layer commonly used in prior art.

According to the present invention, the water-insoluble alkaline compounds may be any water-insoluble alkaline compound commonly used in prior art to improve the stability of acid-unstable compounds. In the embodiments according to the present invention, the water-insoluble alkaline compounds may be selected from, but not limited to, one or more of magnesium hydroxide, aluminum hydroxide, magnesium oxide, magnesium carbonate, calcium carbonate, and calcium hydroxide.

According to the present invention, the water insoluble inert substance that can prevent adhesion of pellets may be selected from one or more of lubricants, flow aids and anti-adherents (i.e. anti-adhesion agents, the same below) conventionally used in pharmacy. In the embodiments according to the present invention, the water insoluble inert substance that can prevent adhesion of pellets may be selected from, but not limited to, silicon dioxide, calcium silicate, colloidal silicon dioxide, aluminum silicate, aluminum calcium silicate, magnesium silicate, sodium stearate, zinc stearate, magnesium stearate, talcum powder, and titanium dioxide, and so on. In an embodiment of Ilaprazole enteric pellets according to the present invention, the first isolating layer of the enteric pellets does not contain one or more of the following water insoluble inert substances that can prevent adhesion of pellets, such as talcum powder, silicon dioxide, titanium dioxide, and magnesium stearate.

In a preferable embodiment of the present invention, the first isolating layer is mainly composed of water-insoluble alkaline compounds and adhesives, and the second isolating layer is mainly composed of water insoluble inert substance that can prevent adhesion of pellets and adhesives. According to the present invention, by adjusting the dosage of water-insoluble alkaline compounds and adhesives contained in the first isolating layer or the dosage of water insoluble inert substance that can prevent adhesion of pellets and adhesives contained in the second isolating layer, it is possible to affect the dissolution rate of the enteric pellet preparations, thus affecting their bioavailability. For example, in a preferable embodiment of Ilaprazole enteric pellet tablets in the present invention, the ratios of the components are shown as follows: if the dosage of Ilaprazole is 5~15 parts by weight, the first isolating layer contains 5~36 parts of adhesives by weight and 5~36 parts of water-insoluble alkaline compounds by weight; the second isolating layer contains 4~26 parts of adhesives by weight and 7~44 parts of water insoluble inert substance by weight that can prevent adhesion of pellets.

The above mentioned adhesives are commonly used in isolating layers in prior art. According to the present invention, the adhesives may be selected from qualified medicinally water-soluble inert compounds or the polymers used as coating films, such as one or more of hydroxypropyl cellulose, hydroxypropyl methyl cellulose, hydroxymethyl cellulose, hydroxyethyl cellulose, carboxymethyl cellulose, methyl cellulose, polyvinyl pyrrolidone, polyvinyl alcohol, polyethylene glycol, syrup, and starch.

### Enteric Layers of Enteric Pellets:

The enteric layers commonly used in enteric pellet preparations in prior art is also applicable to the present invention; for example, a detailed introduction to enteric layers is recorded in CN87103285A (Chinese family patent of US84786505); the inventors used it as a reference and introduced the contents related to the enteric layers in the literature and the relevant contents of the literature cited therein into the present application.

According to the present invention, the enteric layer may contain one or more substances selected from the following groups: acrylic resins, cellulose such as carboxymethyl ethyl cellulose, enteric coating materials such as Opadry, and one or more additives s optionally elected from plasticizers, anti-adherents and lubricants. In the embodiments according to the present invention, the enteric layer may contain acrylic resin enteric coating materials, plasticizers (e.g. polyethylene glycol, glyceryl triacetate, triethyl citrate, phthalate, etc.), and anti-adherents (e.g. talcum powder, glyceryl monostearate, etc.). Wherein, the acrylic resin enteric coating material can be selected from cellulose acetate phthalate, hydroxypropyl methyl cellulose phthalate, solution or dispersion liquid L30D55 of methacrylic acid copolymer, hydroxypropyl methyl cellulose acetate succinate, polyvinyl acetate phthalate, and shellac or any combination thereof. In the prefered embodiment, according to the present invention of itrazole enteric coated pellets, the weight ratio of the enteric coating material contained in the enteric layer to Ilaprazole and/or pharmaceutically acceptable salts thereof is 2~20: 1. In a preferred embodiment of Ilaprazole enteric pellets according to the present invention, the weight ratio of the plasticizer to Ilaprazole and/or its pharmaceutically acceptable salts thereofis 0.6-6:1, preferably 0.8-4:1, and more preferably 1∼2:1.

### Protective Layers of Enteric Pellets:

According to the present invention, Enteric coated pellets can also have a protective layer on the outside of the enteric layer, and preferably the protective layer is closely adjacent to the enteric layer. The protective layer can prevent the adhesion that may occur during the placement process between various semi-finished products/products before or during the production process of their preparations, or during the placement period after their preparations. In addition, the protective layer can effectively increase the dissolution rate of enteric pellets, thereby improving the bioavailability of enteric pellet preparations made from enteric pellets.

In the embodiments according to the present invention, the protective layer may contain adhesives and anti-adherents. The adhesives can be selected from one or more of hydroxypropyl methyl cellulose, hydroxypropyl cellulose, polyvinyl pyrrolidone, polyvinyl alcohol, methyl cellulose, and polyethylene glycol. The anti-adherents can be selected from one or more of talcum powder, magnesium stearate, titanium dioxide, and silicon dioxide.

According to the present invention, the acid resistance of the enteric pellets can be improved by increasing the dosage of the anti-adherents in the protective layer. In the preferred embodiment of Ilaprazole enteric pellets according to the present invention, when Ilaprazole and/or pharmaceutically acceptable saltsthereof,is 5-15 parts by weight, the dosage of anti-adherents is 0.5∼5 parts by weight.

According to the fourth aspect of the present invention, a method for preparation of enteric pellets is provided. The preparation method comprises at least the following steps: 1) preparing the pellet core comprising Ilaprazole and/or its pharmaceutically acceptable salts thereof and the first excipient; 2) coating the first isolation layer, and then coating the second isolation layer; and 3) coating the enteric layer.

Preferably, step 2) includes preparing the first suspension solution comprising water-insoluble alkaline compounds and devoid of water soluble alkaline compounds and the water insoluble inert substance that can prevent adhesion of pellets, and coating the first suspension on the pellet core obtained in step 1); prepare the second suspension solution without alkaline compounds, and it is coated as the second isolating layer, preferably as the second isolating layer closely adjacent to the enteric layer.

Preferably, in step 1), the first excipient interacts with the water-insoluble alkaline compounds contained in the first isolating layer, thereby achieving the storage stability of Ilaprazole and/or pharmaceutically acceptable salts thereof.

Preferably, the preparation method for enteric pellets also includes step 4): coating the protective layer.

In the embodiments according to the present invention, the preparation method for enteric pellets can comprise one or more of the following steps:
1) This step involves coating the blank pellet core with a drug loading layer, for example, using the fluidized bed method, to prepare the pellet core; the drug loading layer includes Ilaprazole and/or pharmaceutically acceptable salts thereof, the alkaline compounds as the first excipient, and the adhesives;

step 2) involves coating at least the first suspension solution and the second suspension solution respectively, on the pellet core obtained in step 1) using a fluid bed method, for example, in an inward-to-outward manner; wherein, the first suspension solution contains water-insoluble alkaline compounds, but lacks water-soluble alkaline compounds and the water insoluble inert substance that can prevent adhesion of pellets, forming the first isolating layer; the second suspension solution does not contain alkaline compounds, forming the second isolating layer; the isolation pellets are prepared in this way.
step3) involves preparing the enteric layer suspension solution with the enteric coating materials and one or more substances selected from plasticizers, anti-adherents, lubricants and emulsifiers. The enteric layer suspension solution is then coated on the isolating pellets obtained in step 2), using a fluid bed method, for example, to prepare the enteric pellets. Preferably, the abovementioned preparation method for enteric pellets can further include step 4): adding one or more adhesives selected from hydroxypropyl cellulose, hydroxypropyl methyl cellulose, methyl cellulose, polyvinyl pyrrolidone, polyvinyl alcohol, carboxymethyl cellulose, and polyethylene glycol into purified water to prepare the coating solution of protective layers, then it is coated on the enteric pellets obtained in step 3), using the fluidized bed method, for example , to prepare the enteric pellets with protective layers.

Using the dry suspension as an example, the preparation methods and the property test experiments for the enteric pellets according to the present invention and the dry suspension comprising the enteric pellets are described below.

### Examples

### Example 1: Preparation of pellets

First, in this example, the enteric pellets A, D, and E according to the present invention are prepared, by using Ilaprazole and/or its pharmaceutically acceptable salt as an example.

### (I) Preparation prescription for the component layers of pellets:

### 1.1 Preparation of drug-containing pellets (i.e. pellet core W) and its application rate

(1) 150g of hydroxypropyl cellulose-SSL and 4g of polysorbate 80 are weighed and dissolved in 3000g of purified water to obtain hydroxypropyl cellulose adhesive solution, then 100g of magnesium hydroxide is added into the adhesive solution and dispersed at high shear of 10000 rpm for 5 min, next 100g of Ilaprazole with particle size D90 of 46.8µm is dispersed into the adhesive solution comprising magnesium hydroxide and dispersed uniformly at high shear rate of 10000 rpm; Ilaprazole suspension solution is sprayed onto 100g of sucrose pellet cores using GLATT GPCG-1 fluidized bed to produce the drug-containing pellet W1.

The process parameters of the fluidized bed are shown as follows:

| Parameters | Preheating | Drug Application | Drying |
|---|---|---|---|
| Air intake rate (m³/min) | 45 | 50-80 | 60 |
| Air intake temperature (°C) | 40-50 | 40-50 | 40-50 |
| Temperature of material (°C) | 38-40 | 35-40 | 35-40 |
| Atomization pressure (bar) | 0.1 | 0.1-0.2 | 0.1 |
| Spraying rate (g/min) | 0.5-4 | | |

Wherein, the drug application rate of drug-containing pellet W1 = (Actual content of drug-containing pellets) / (Theoretical content of drug-containing pellets) ×100% = 95.6%

(2) 180g of polyvinyl pyrrolidone K30 and 6g of polysorbide 80 are weighed and dissolved in 3000g of purified water, and 50g of magnesium oxide is added and dispersed uniformly with high shear at 10000 rpm to obtain the adhesive comprising magnesium oxide; then 150g of Ilaprazole is dispersed into the adhesive comprising magnesium oxide and dispersed uniformly with high shear at 10000 rpm; Ilaprazole suspension solution is sprayed onto 100g of mannitol pellet cores using GLATT GPCG-1 fluidized bed toproduce the drug-containing pellet W4.

The process parameters of the fluidized bed are shown as follows:

| Parameters | Preheating | Drug Application | Drying |
|---|---|---|---|
| Air intake rate (m³/min) | 50 | 50-70 | 60 |
| Air intake temperature (°C) | 40-50 | 45-55 | 45-55 |
| Temperature of material (°C) | 38-40 | 35-40 | 35-40 |
| Atomization pressure (bar) | 0.1 | 0.1-0.2 | 0.1 |
| Spraying rate (g/min) | 0.5-4 | | |

Wherein, the drug application rate of drug-containing pellet W4 = (Actual content of drug-containing pellets) / (Theoretical content of drug-containing pellets) ×100% = 93.3%

(3) 80g of hydroxypropyl methyl cellulose E5 and 6g of polysorbate 80 are weighed and dissolved in 1600g of purified water, and 50g of magnesium hydroxide is added and dispersed uniformly with high shear at 10000 rpm to obtain the adhesive comprising magnesium hydroxide; then 150g of Ilaprazole is dispersed into the adhesive comprising magnesium hydroxide and dispersed uniformly with high shear at 10000 rpm; Ilaprazole suspension solution is sprayed onto 150g of sucrose pellet cores using GLATT GPCG-1 fluidized bed to produce the drug-containing pellet W5.

The process parameters of the fluidized bed are shown as follows:

| Parameters | Preheating | Drug Application | Drying |
|---|---|---|---|
| Air intake rate (m³/min) | 50 | 50-70 | 60 |
| Air intake temperature (°C) | 40-50 | 45-55 | 45-55 |
| Temperature of material (°C) | 38-40 | 35-40 | 35-40 |
| Atomization pressure (bar) | 0.1 | 0.1-0.2 | 0.1 |
| Spraying rate (g/min) | 0.5-4 | | |

Wherein, the drug application rate of drug-containing pellet W5 = (Actual content of drug-containing pellets) / (Theoretical content of drug-containing pellets) ×100% = 94.1%

### 1.2 Preparation of the isolating layer (G)

23g of hydroxypropyl cellulose-SSL is weighed and dissolved in 460g of purified water, and then 23g of magnesium carbonate is added and dispersed uniformly with high shear at 10000 rpm to prepare the prescription for the first isolating layer. 17.2g of hydroxypropyl cellulose-SSL is weighed and dissolved in 344g of purified water, and 28.8g of talcum powder is added and dispersed uniformly with high shear at 10000 rpm to prepare the prescription for the second isolating layer. The two layers of isolating suspension solution are sprayed respectively onto 90g of corresponding pellet cores using GLATT GPCG-1 fluidized bed.

The process parameters of the fluidized bed are shown as follows:

| Parameters | Preheating | Isolating Coating | Drying |
|---|---|---|---|
| Air intake rate (m³/min) | 50 | 50-60 | 60 |
| Air intake temperature (°C) | 40-50 | 45-55 | 45-55 |
| Temperature of material (°C) | 38-40 | 35-40 | 35-40 |
| Atomization pressure (bar) | 0.1 | 0.1-0.2 | 0.1 |
| Spraying rate (g/min) | 0.5-3.5 | | |

### 1.3 Preparation of the enteric layer (C)

Enteric layers prescription C (Units: g)

| | |
|---|---|
| Ilaprazole isolating pellets | 92 |
| Eudragit L30D-55 | 223.8 |
| Talcum powder | 3.4 |
| Triethyl citrate | 20.1 |
| Purified water | 447.6 |

### Process of preparation:

20.1g of triethyl citrate is weighed and dissolved in 447.6g of purified water, and 3.4g of talcum powder is added and dispersed uniformly with high shear at 10000 rpm; and then the solution is stirred with 223.8g of Eudragit L30D-55 for 45 min for standby, and the enteric coating solution is sprayed onto the isolating pellets in GLATT GPCG-1 fluidized bed.

The specific process parameters are shown as follows:

| Parameters | Preheating | | Enteric Coating | | Drying | |
|---|---|---|---|---|---|---|
| Air intake rate (m³/min) | | 50 | | 50-80 | | 60 |
| Air intake temperature (°C) | | 35-30 | | 33-42 | | 35-45 |
| Temperature of material (°C) | | 25-30 | | 25-30 | | 30-35 |
| Atomization pressure (bar) | | 0.1 | | 0.15-0.25 | | 0.1 |
| Spraying rate (g/min) | | 0.5-3 | | | | |

### 1.4 Preparation of the protective layer (B)

### 1) Protective layers prescription B1 (Units: g)

| | | |
|---|---|---|
| Ilaprazole enteric pellets | | 100 |
| Hydroxypropyl methyl cellulose E5 | | 2.5 |
| Magnesium stearate | | 1.2 |
| Purified water | | 50 |

### Process of preparation:

2.5g of hydroxypropyl methyl cellulose E5 in prescription dosage is weighed and dissolved in 50g of purified water, and then 1.2g of magnesium stearate is added and dispersed uniformly with high shear at 5000 rpm to prepare the coating solution of protective layers; the coating solution of protective layers is sprayed onto the enteric pellets in GLATT GPCG-1 fluidized bed.

The specific process parameters are shown as follows:

| Parameters | Preheating | Isolating Coating | Drying |
|---|---|---|---|
| Air intake rate (m³/min) | 50 | 50-60 | 60 |
| Air intake temperature (°C) | 40-50 | 45-55 | 45-55 |
| Temperature of material (°C) | 38-40 | 35-40 | 35-40 |
| Atomization pressure (bar) | 0.1 | 0.1-0.2 | 0.1 |
| Spraying rate (g/min) | 0.5-3.5 | | |

### 2) Protective layers prescription B4 (Unit: g)

| | | |
|---|---|---|
| Ilaprazole enteric pellets | | 100 |
| Hydroxypropyl cellulose-SSL | | 2.5 |
| Talcum powder | | 1.2 |
| Purified water | | 50 |

### Process of preparation:

2.5g of hydroxypropyl cellulose-SSL in prescription dosage is weighed and dissolved in 50g of purified water, and then 1.2g of talcum powder is added and dispersed uniformly with high shear at 5000 rpm to prepare the coating solution of protective layers. The coating solution of protective layers is sprayed onto the enteric pellets in GLATT GPCG-1 fluidized bed.

Please see the abovementioned protective layers prescription B1 for the specific process parameters.

### 3) Protective layers prescription B5 (Units: g)

| | | |
|---|---|---|
| Ilaprazole enteric pellets | | 100 |
| Hydroxypropyl cellulose-SSL | | 2.5 |
| Titanium dioxide | | 1.2 |
| Purified water | | 50 |

### Process of preparation:

2.5g of hydroxypropyl cellulose-SSL in prescription dosage is weighed and dissolved in 50g of purified water, and then 1.2g of titanium dioxide is added and dispersed uniformly with high shear at 5000 rpm to prepare the coating solution of protective layers. The coating solution of protective layers is sprayed onto the enteric pellets in GLATT GPCG-1 fluidized bed.

Please see the abovementioned protective layers prescription B1 for the specific process parameters.

### (II) Preparation of enteric pellets:

According to the prescriptions for the component layers of enteric pellets as set forth in Section (I) of this example, the enteric pellets A, D and E according to the present invention are prepared as shown in Table 1 below.

**Table 1: Prescription Components of Enteric Pellets in the present invention**

| | Pellets A | Pellets D | Pellets E |
|---|---|---|---|
| drug-containing pellets | W1 | W4 | W5 |
| Isolating layers | G2 | G2 | G2 |
| Enteric layers | C1 | C1 | C1 |
| Protective layers | B1 | B4 | B5 |
| Drug loading capacity of drug-containing pellets (%) | 95.6 | 93.3 | 94.1 |

Wherein, Wₓ the drug application rate of drug-containing pellets = (Actual content of drug-containing pellets) / (Theoretical content of drug-containing pellets) × 100%

### Example 2: Preparation of pellets dry suspension

In this example, first the dry suspension granules are prepared, and then different pellets are added to prepare the dry suspension comprising pellets.

### 2.1 Preparation of dry suspension A-I

### 1) Preparation of dry suspension granules

| Prescription | Dosage |
|---|---|
| Carrageenan | 25g |
| Hydroxypropyl cellulose LF | 10g |
| Crosslinked povidone | 25g |
| Mannitol | 700g |
| Glucose | 250g |
| Malic acid | 4g |
| Chitosan | 25g |
| Purified water | 190g |

Preparation method: 10g of hydroxypropyl cellulose LF is weighed and dissolved in 190g of purified water, and the prescription dosage of carrageenan, crosslinked povidone, chitosan, mannitol, glucose, and malic acid are weighed and added into HLSH2-6 wet mixing granulators. The mixture is stirred at 400 rpm for 4 min, and then hydroxypropyl cellulose LF is dissolved and added into the mixer for further stirring for 3 min. The shearing is conducted at 800 rpm for 1 min before discharging, to obtain the wet granules; the wet granules are dried and sieved through a 1.2mm mesh to obtain the dry suspension granules I.

### 2) Preparation of dry suspension

94.5mg of Ilaprazole enteric pellets A and 1.5g of the abovementioned dry suspension granules are mixed to obtain the dry suspension A-I.

### 2.2 Preparation of dry suspension D-II

### 1) Preparation of dry suspension granules

| Prescription | Dosage |
|---|---|
| Arabic gum | 20g |
| Sodium alginate | 10g |
| Hydroxypropyl methyl cellulose E5 | 15g |
| Crosslinked sodium carboxymethyl cellulose | 30g |
| Mannitol | 600g |
| Sucrose | 400g |
| Citric acid | 3.5g |
| Chitosan | 10g |
| Purified water | 210g |

Preparation methods: The prescription dosage of Arabic gum, sodium alginate, hydroxypropyl methyl cellulose E5, crosslinked sodium carboxymethyl cellulose, mannitol, sucrose, citric acid, and chitosan are weighed and delivered into HLSH2-6 wet mixing granulator, stirred and mixed at 400 rpm for 5 min, then the purified water is added slowly while stirring, and the solution is further stirred for 5 min. The shearing is conducted at 700 rpm for 1 min to prepare the wet granules, which are discharged, dried, and granulated with 1.0mm mesh to obtain the dry suspension granules II.

### 2) Preparation of dry suspension

67.4mg of Ilaprazole enteric pellets D and 2.5g of the abovementioned dry suspension granules are mixed, then bottled to obtain Ilaprazole enteric dry suspension D-II in the specification of 5mg.

### 2.3 Preparation of dry suspension E-III

### 1) Preparation of dry suspension granules III

| Prescription | Dosage |
|---|---|
| Pectin | 30g |
| Sodium alginate | 5g |
| Polyvinyl pyrrolidone K30 | 10g |
| Crosslinked povidone | 35g |
| Maltose | 200g |
| Xylitol | 900g |
| Ascorbic acid | 9g |
| Chitosan | 35g |
| Purified water | 190g |

Preparation methods: 10g of polyvinyl pyrrolidone K30 is weighed and dissolved in 190g of purified water, and the prescription dosage of pectin, sodium alginate, crosslinked povidone, maltose, xylitol, ascorbic acid, and chitosan are weighed and delivered into HLSH2-6 wet mixing granulator. The mixture was stirred at 400 rpm for 4 min, then polyvinyl pyrrolidone solution is added into the mixer and further stirred for 4 min; the shearing is conducted at 800 rpm for 1 min before discharging to obtain the wet granules; the wet granules are dried and granulated with 1.2mm mesh to obtain the dry suspension granules III.

### 2) Preparation of dry suspension

60.5mg of Ilaprazole enteric pellets E and 2.0g of the abovementioned dry suspension granules are mixed, then bottled to obtain Ilaprazole enteric dry suspension E-III in the specification of 5mg.

### 2.4 Preparation of dry suspension A-VI

### 1) Preparation of dry suspension granules VI

| Prescription | Dosage |
|---|---|
| Xanthan gum | 25g |
| Hydroxypropyl methyl cellulose E5 | 8g |
| Crosslinked sodium carboxymethyl cellulose | 20g |
| Mannitol | 600g |
| Sucrose | 250g |
| Citric acid | 4g |
| Chitosan | 5g |
| Purified water | 220g |

Preparation methods: 8g of hydroxypropyl methyl cellulose E5 is weighed and dissolved in 220g of purified water, and the prescription dosage of xanthan gum, crosslinked sodium carboxymethyl cellulose, chitosan, mannitol, sucrose, and citric acid are weighed and delivered into HLSH2-6 wet mixing granulator , The mixture was stirred at 400 rpm for 4 min, then hydroxypropyl methyl cellulose E5 solution is added into the mixer and further stirred for 3 min; the shearing is conducted at 1000 rpm for 1 min before discharging to make the wet granules; the wet granules are dried and granulated with 1.2mm mesh to obtain the dry suspension granules VI.

### 2) Preparation of dry suspension

94.5mg of Ilaprazole enteric pellets A and 1.6g of the abovementioned dry suspension granules for are mixed, then bottled to obtain Ilaprazole enteric dry suspension A-VI in the specification of 5mg.

### 2.5 Preparation of dry suspension A-VII

### 1) Preparation of dry suspension granules VII

| Prescription | Dosage |
|---|---|
| Xanthan gum | 30g |
| Hydroxypropyl methyl cellulose VLV | 18g |
| Crosslinked sodium carboxymethyl cellulose | 30g |
| Mannitol | 600g |
| Sucrose | 260g |
| Citric acid | 6g |
| Chitosan | 3g |
| Purified water | 280g |

Preparation methods: 18g of hydroxypropyl methyl cellulose VLV is weighed and dissolved in 280g of purified water, and the prescription dosage of xanthan gum, crosslinked sodium carboxymethyl cellulose, chitosan, mannitol, sucrose, and citric acid are weighed and delivered into HLSH2-6 wet mixing granulator. The mixture was stirred at 400 rpm for 4 min, then hydroxypropyl methyl cellulose VLV solution is added into the mixer and further stirred for 3 min; the shearing is conducted at 800 rpm for 1 min before discharging to prepare the wet granules; the wet granules are dried and granulated with 1.2mm mesh to obtain the dry suspension granules VII.

### 2) Preparation of dry suspension

94.5mg of Ilaprazole enteric pellets A and 2.0g of the abovementioned dry suspension granules are mixed, then bottled to obtain Ilaprazole enteric suspension A-VII in the specification of 5mg.

### 2.6 Preparation of dry suspension A-VIII

### 1) Preparation of dry suspension granules VIII

| Prescription | Dosage |
|---|---|
| Xanthan gum | 20g |
| Hydroxypropyl methyl cellulose VLV | 18g |
| Crosslinked sodium carboxymethyl cellulose | 30g |
| Mannitol | 600g |
| Sucrose | 260g |
| Citric acid | 6g |
| Chitosan | 40g |
| Purified water | 280g |

Preparation methods: 18g of hydroxypropyl methyl cellulose VLV is weighed and dissolved in 280g of purified water, and the prescription dosage of xanthan gum, crosslinked sodium carboxymethyl cellulose, chitosan, mannitol, sucrose, and citric acid are weighed and delivered into HLSH2-6 wet mixing granulator. The mixture was stirred at 400 rpm for 4 min, then hydroxypropyl methyl cellulose VLV solution is added into the mixer and further stirred for 3 min; the shearing is conducted at 800 rpm for 1 min before discharging to prepare the wet granules; the wet granules are dried and granulated with 1.2mm mesh, to obtain the dry suspension granules VIII.

### 2) Preparation of dry suspension

94.5mg of Ilaprazole enteric pellets A and 1.8g of the abovementioned dry suspension granules are mixed, then bottled to obtain Ilaprazole enteric dry suspension A-VIII in the specification of 5mg.

### 2.7 Preparation of dry suspension A-IX

### 1) Preparation of dry suspension granules IX

| Prescription | dosage |
|---|---|
| Xanthan gum | 20g |
| Hydroxypropyl methyl cellulose VLV | 20g |
| Crosslinked sodium carboxymethyl cellulose | 30g |
| Mannitol | 660g |
| Sucrose | 260g |
| Citric acid | 8g |
| Chitosan | 60g |
| Purified water | 300g |

Preparation methods: 20g of hydroxypropyl methyl cellulose VLV is weighed and dissolved in 300g of purified water, and the prescription dosage of xanthan gum, crosslinked sodium carboxymethyl cellulose, chitosan, mannitol, sucrose, and citric acid are weighed and delivered into HLSH2-6 wet mixing granulator and stirred and mixed at 400 rpm for 4 min, and then hydroxypropyl methyl cellulose VLV solution is added into the mixer and further stirred for 3 min; the shearing is conducted at 800 rpm for 1 min before discharging to prepare the wet granules; the wet granules are dried and granulated with 1.2mm mesh to obtain the dry suspension granules IX.

### 2) Preparation of dry suspension

94.5mg of Ilaprazole enteric pellets A and 1.8g of the abovementioned dry suspension granules are mixed, then bottled to obtain Ilaprazole enteric dry suspension A-IX in the specification of 5mg.

### 2.8 Preparation of dry suspension in comparative example

### 1) Prescription of contrastive dry suspension granules V (Units: g)

| Components | Weight (g) |
|---|---|
| Xanthan gum | 20 |
| Chitosan | 1400 |
| CCNA | 75 |
| Aspartame | 15 |

Preparation methods: Xanthan gum, chitosan, CCNA and aspartame are mixed uniformly, then added into the dry granulator for granulation. The process parameters are shown as follows: Pinch roller spacing of 0.2mm, feeding speed of 30 rpm, pinch roller rotation speed of 5 rpm, and granulating rotation speed of 10 rpm. After the granulation is completed, they are sieved for granulation with the particle size controlled within 0.5-0.7mm to obtain the dry suspension granules V.

### 2) Preparation of dry suspension

60.5mg of Ilaprazole enteric pellets E and 2.0g of the abovementioned dry suspension granules V are mixed, then bottled to obtain Ilaprazole enteric dry suspension E-V in the specification of 5mg.

### Example 3: Performance measurement of dry suspension granules

### 3.1 Viscosity measurement of dry suspension granules

### 3.1.1 Dry suspension granules I in the present invention

15mL of water is added into 1.5g of dry suspension granules I, and the suspension is stirred for 60s; a calibrated pH meter is used to measure the pH value at 3.9, and then the viscosity of the suspension gel is measured continuously.

### 3.1.2 Dry suspension granules II in the present invention

25mL of water is added into 2.5g of dry suspension granules II, and the suspension is stirred for 60s; a calibrated pH meter is used to measure the pH value at 3.8, and then the viscosity of the suspension gel is measured continuously.

### 3.1.3 Dry suspension granules III in the present invention

20mL of water is added into 2.0g of dry suspension granules III, and the suspension is stirred for 60s; a calibrated pH meter is used to measure the pH value at 4.0, and then the viscosity of the suspension gel is measured continuously.

### 3.1.4 Dry suspension granules VI in the present invention

20mL of water is added into 2.0g of dry suspension granules VI, and the suspension is stirred for 60s; a calibrated pH meter is used to measure the pH value at 4.0, and then the viscosity of the suspension gel is measured continuously.

### 3.1.5 Dry suspension granules VII in the present invention

20mL of water is added into 2.0g of dry suspension granules VII, and the suspension is stirred for 60s; a calibrated pH meter is used to measure the pH value at 3.9, and then the viscosity of the suspension gel is measured continuously.

### 3.1.6 Dry suspension granules VIII in the present invention

20mL of water is added into 2.0g of dry suspension granules VIII, and the suspension is stirred for 60s; a calibrated pH meter is used to measure the pH value at 3.9, and then the viscosity of the suspension gel is measured continuously.

### 3.1.7 Dry suspension granules IX in the present invention

20mL of water is added into 2.0g of dry suspension granules IX, and the suspension is stirred for 60s; a calibrated pH meter is used to measure the pH value at 4.0, and then the viscosity of the suspension gel is measured continuously.

### 3.1.8 Dry suspension granules IV in comparative example

As a sample of prior art, NEXIUM^{®} (ORAL SUSPENSION, in the specification of 40mg) from AstraZeneca is used; the enteric pellets comprising Esomeprazole magnesium are removed from the granules of the product, and 15mL of purified water is added into the residual powder, and the suspension is stirred for 60s, and then the viscosity of the suspension gel is measured continuously.

### 3.1.9 Dry suspension granules V in comparative example

20mL of water is added into 2.0g of dry suspension granules V in comparative example, and the suspension is stirred for 60s; a calibrated pH meter is used to measure the pH value at 8.0, and then the viscosity of the suspension gel is measured continuously.

Equipments: BROOKFIELD DV2T viscometer, small sample adapter SSA, and MV1Y flag-shaped impeller blade rotors.

See Table 2 and Figure 1 below for the viscosity measurement results of the abovementioned dry suspension granules:

**Table 2: Viscosity Measurement Results of Dry suspension granules**

| Time taken to reach % of maximum viscosity | | |
|---|---|---|
| Percentage in maximum viscosity | >75% | >90% |
| Dry suspension granules I in the present invention (n=6) | Average=5.4 min | Average=8.8 min |
| | Minimum=4.6 min | Minimum=7.5 min |
| | Maximum=8.3 min | Maximum=11.6 min |
| Dry suspension granules II in the present invention (n=6) | Average=5.6 min | Average=10.5 min |
| | Minimum=5.0 min | Minimum=7.2 min |
| | Maximum=9.7 min | Maximum=12.3 min |
| Dry suspension granules III in the present invention (n=6) | Average=6.5 min | Average=9.4 min |
| | Minimum=5.1 min | Minimum=8.3 min |
| | Maximum=8.8 min | Maximum=13.8 min |
| Dry suspension granules VI in the present invention (n=6) | Average= 7.2 min | Average=8.9 min |
| | Minimum=6.5 min | Minimum=7.9 min |
| | Maximum=8.9 min | Maximum=9.6 min |
| Dry suspension granules VII in the present invention (n=6) | Average=7.9 min | Average=8.6 min |
| | Minimum=6.8 min | Minimum=6.3 min |
| | Maximum=9.8 min | Maximum=10.5 min |
| Dry suspension granules VIII in the present invention (n=6) | Average=18.9 min | Average=19.5 min |
| | Minimum=17.2 min | Minimum=16.9 min |
| | Maximum=22.3 min | Maximum=24.2 min |
| Dry suspension granules IX in the present invention (n=6) | Average=19.8 min | Average=22.5 min |
| | Minimum=14.6 min | Minimum=21.5 min |
| | Maximum=22.1 min | Maximum=24.6 min |
| Dry suspension granules IV in comparative example in prior art NEXIUM^{®} (n=6) | Average=9.7 min | Average=13.5 min |
| | Minimum=7.2 min | Minimum=9.1 min |
| | Maximum=11.5 min | Maximum=20.3 min |
| Dry suspension granules V in contract (n=6) | Average=19.7 min | Average=23.5 min |
| | Minimum=15.2 min | Minimum=19.1 min |
| | Maximum=21.5 min | Maximum=30.3 min |

It can be seen from Table 2 and Figure 1 that the dry suspension granules I-III and VI-VIII in the present invention can reach the maximum viscosity faster than the dry suspension granules IV in prior art; However, compared to the suspension V, due to the large dosage of chitosan, it will take a significantly longer time to reach the maximum viscosity after water is added; furthermore, the dry suspension granules V in comparative example will become alkaline after being added into water; as a result, the enteric coating of Ilaprazole enteric pellets will be dissolved.

### 3.2 Test on the time required for maintaining suspension of Ilaprazole pellets in the suspension

(1) In the example 2.1, 15mL of water is added into the dry suspension A-I, and stirred at constant speed and at 30s, the timer is started to observe the time taken for all the enteric pellets to suspend.
(2) In the example 2.2, 25mL of water is added into the dry suspension D-II, the suspension is stirred at constant speed; and at 30s, the timer is started to observe the time taken for all the enteric pellets to suspend.
(3) In the example 2.3,20mL of water is added into the dry suspension E-III, the suspension is stirred at constant speed; and at 30s, the timer is started to observe the time taken for all the enteric pellets to suspend.
(4) 15mL of purified water is added into NEXIUM^{®} (ORAL SUSPENSION, in the specification of 40mg) from AstraZeneca, the suspension is stirred at constant speed; and at 30s, the timer is started to observe the time taken for all the enteric pellets to suspend.
(5)In the example 2.4, 20mL of water is added into the dry suspension E-V in comparative example, the suspension is stirred at constant speed; at30s, the timer is started to observe the time taken for all the enteric pellets to suspend.

See Table 20 belowfor the results of the abovementioned tests:

**Table 3: Test Results on the Time Required to Maintain Suspension of Ilaprazole Pellets in Suspension**

| Samples | Time for pellets to maintain suspension |
|---|---|
| Dry Suspension A-I in the present invention | 38s |
| Dry Suspension D-II in the present invention | 45s |
| Dry Suspension E-III in the present invention | 33s |
| Dry Suspension IV in prior art, NEXIUM^{®} | 119s |
| Dry Suspension E-V in comparative example | 735s |

It can be seen from Table 3 that dry suspensions A-I, D-II and E-III in the present invention can form suspension gel in a shorter time than the prior art; the time required for the dry suspended pellets in dry suspension E-V in comparative example to maintain suspension needs to be up to 735s, and the suspension velocity is affected due to the too high gel strength.

### 3.3 Suspension-sedimentation time of pellets in suspension gel

(1) 100 Ilaprazole enteric pellets A and 15mL of water are added into 1.5g of dry suspension granules I of the present invention, and the suspension is stirred for 150s until the enteric pellets are completely suspended.; and then poured into a 15mL volumetric cylinder, the number of enteric pellets within the measuring range of 0-5mL, 5-10mL, and 10-15mL is counted at 1h intervals for 12 h. Ten groups of tests are conducted simultaneously. The average proportion of the pellets within the measuring range of 0-5mL, 5-10mL, and 10-15mL is calculated (see the curve of dry suspension granules I in Figures 2~4).
(2) 100 Ilaprazole enteric pellets D and 15mL of water are added into 1.5g of dry suspension granules II of the present invention, and the suspension is stirred for 150s until the enteric pellets are completely suspended. and then poured into a 15mL volumetric cylinder, the number of enteric pellets within the measuring range of 0-5mL, 5-10mL, and 10-15mL is counted at 1h intervals for 12 h. Ten groups of tests are conducted simultaneously. The average proportion of the pellets within the measuring range of 0-5mL, 5-10mL, and 10-15mL is calculated (see the curve of dry suspension granules II in Figures 2~4).
(3) 100 Ilaprazole enteric pellets E and 15mL of water are added into 1.5g of dry suspension granules III of the present invention, and stirred for 150s until the pellets are completely suspended.; and then poured into a 15mL volumetric cylinder, the number of enteric pellets within the measuring range of 0-5mL, 5-10mL, and 10-15mL is counted at 1h intervals for 12 h. Ten groups of tests are conducted simultaneously. The average proportion of the pellets within the measuring range of 0-5mL, 5-10mL, and 10-15mL is calculated (see the curve of dry suspension granules III in Figures 2~4).
(4) As the dry suspension granules IV, it isa product in prior art, i.e. NEXIUM^{®} (ORAL SUSPENSION, in the specification of 40mg) from AstraZeneca; the enteric pellets containing Esomeprazole magnesium are completely removed from the dry suspension granules of the product, then 100 Esomeprazole magnesium enteric pellets are added to the granules, 15mL of water are added , , and stirred for 150s until the pellets are fully suspended fully; and then poured into a 15mL volumetric cylinder, the number of enteric pellets within the measuring range of 0-5mL, 5-10mL, and 10-15mL is counted at 1h intervals for 12h.. Ten groups of tests are conducted simultaneously. The average proportion of the pellets within the measuring range of 0-5mL, 5-10mL, and 10-15mL is calculated (see the curve of dry suspension granules IV in prior art, NEXIUM^{®} in Figures 2~4).
(5) 100 Esomeprazole magnesium enteric pellets of NEXIUM^{®} (ORAL SUSPENSION) from AstraZeneca are added into 1.5g of dry suspension granules I in the present invention, 15mL of water are added into 1.5g of dry suspension granules I of the present invention, stirred for 150s until the enteric pellets are fully suspended and then poured into a 15mL volumetric cylinder, the number of enteric pellets within the measuring range of 0-5mL, 5-10mL, and 10-15mL are counted at 1h intervals for 12h. Ten groups of tests are conducted simultaneously. The average proportion of the pellets within the measuring range of 0-5mL, 5-10mL, and 10-15mL is calculated (see the curve of dry suspension granules Ia in Figures 2~4).
(6) 100 Celphere CP-507 microcrystalline cellulose blank pellet cores (particle size ranging from 500µm to 700µm) from Asahi Kasei and 15mL of water are added into 1.5g of dry suspension granules II of the present invention, and stirred for 150s until the enteric pellets are fully suspended then poured into a 15mL volumetric cylinder, the number of enteric pellets within the measuring range of 0-5mL, 5-10mL, and 10-15mL are counted at 1h intervals for 12h. Ten groups of tests are conducted simultaneously. The average proportion of the pellets within the measuring range of 0-5mL, 5-10mL, and 10-15mL is calculated (see the curve of dry suspension granules IIa in Figures 2~4).

The results of the above tests are shown in Figure 2~Figure 4,which show thatwithin the measuring range of 0-5ml, the proportion of the enteric pellets in the suspension formed by the dry suspension granules IV in prior art is higher than that in the suspension formed by the dry suspension granules I, II or III according to the present invention; furthermore, as the time goes by, the proportion of the enteric pellets in the suspension formed by the dry suspension granules IV increases significantly. In contrast, within the measuring range of 10-15ml, the proportion of the enteric pellets in the suspension formed by the dry suspension granules IV is lower than that in the suspension formed by the dry suspension granules I, II or III; and over time, the proportion of the enteric pellets in the suspension formed by the dry suspension granules IV becomes lower. These facts indicate that the suspension/suspension gel prepared with the dry suspension granules according to the present inventionallow the enteric pellets to be suspended for a longer time and are less likely to sediment.

### 3.4 Measurement of acid resistance of Ilaprazole enteric pellets in different dry suspensions

The acid resistance of Ilaprazole enteric pellets in the dry suspension according to the present invention and the dry suspension in contrast is measured by reference to the following measurement method. Please see the results in Table 4 below:
Measurement methods:
Take the dry suspension containing enteric pellets, with 0.1mol/L hydrochloric acid solution (9.0mL of concentrated hydrochloric acid is added with water to the volume of 1000mL) as the dissolution medium, and the rotation speed of 100 rpm. After 120 min, the dissolution cup is taken out and the solution is filtered with suction filter. The residual pellets are collected into a 50mL volumetric flask; 20mL of 0.05mol/L sodium hydroxide solution is added, placed in an oscillator for agitation (250 rpm) for 20 min; 30mL of methanol is added and ultrasonicated for 10 min, diluted with water to the scale , shaken well, centrifuged, and the supernatant was taken. and the supernatant is taken after shaking well and centrifuging. The content of residual drug in the pellets is measured, i.e. the acid resistance of the sample.

**Table 4: Results of Acid Resistance assay of Ilaprazole Enteric Pellets in Different Dry Suspensions**

| Duration | Dry Suspension A-I | Dry Suspension D-II | Dry Suspension E-III | Dry Suspension E-V |
|---|---|---|---|---|
| 0 day | 97.5% | 95.5% | 96.4% | 95.6% |
| 1 month | 95.6% | 96.1% | 95.8% | 96.8% |
| 3 months | 96.4% | 95.7% | 96.5% | 95.4% |
| 6 months | 94.5% | 96.0% | 97.5% | 94.8% |

### 3.5 Measurement of dissolution of Ilaprazole enteric pellets in different dry suspensions

The dissolution rate of Ilaprazole enteric pellets in the dry suspension according to the present invention and the dry suspension in contrast is measured by reference to the measurement methods for dissolution and release (for the general methods, see method 1 of the second Method 2 in the General Rule 0931 of Chinese Pharmacopoeia 2015 Edition Volume IV). The results of measurement are shown in Table 5 below:
Measurement methods:
Take the sample to be tested, with 300mL of 0.1mol/L hydrochloric acid solution (9.0mL of hydrochloric acid is added with water to the volume of 1000mL) as the dissolution medium, and the rotation speed of 100 rpm, and the operations are carried out according to the method for 120 min; then700mL of 0.086mol/L disodium hydrogen phosphate solution preheated to 37±0.5°C (30.8g of disodium hydrogen phosphate and 7g of Tween 80 are taken, and water is added to the volume of 1000mL) is added into the dissolution cups, and then mixed uniformly while the rotation speed remains unchanged. The operations are continued to carry out according to the method, after 45 min the sample is taken.

Test sample solution: An appropriate amount of the dissolved solution is taken and filtered, 5mL of filtrate is measured accurately, and 1mL of 0.15mol/L sodium hydroxide solution is added accurately and immediately, and then the solution is shaken well and filtered; the filtrate is taken as the test sample solution.

Reference substance solution: About 10mg of Ilaprazole reference substance is weighed accurately and put into a 20mL volumetric flask, and appropriate amount of acetonitrile is added to dissolve it, and the solution is diluted to the scale with acetonitrile and shaken well; 1mL of the solution is measured accurately and put into a 100mL volumetric flask, and then diluted to the scale with phosphate buffer solution (pH 6.8) (700mL of 0.086mol/L disodium hydrogen phosphate solution and 300mL of 0.1mol/L hydrochloric acid solution are mixed uniformly to obtain the phosphate buffer solution) and shaken well; 5mL of this solution is measured accurately, and 1mL of 0.05mol/L sodium hydroxide solution is added accurately and immediately, and then the solution is shaken well and filtered; the subsequent filtrate is taken as the reference substance solution.

**Table 5: Measurement Results of Dissolution Rate of Ilaprazole Enteric Pellets in Different Dry Suspensions**

| T/min | Dry Suspension A-I | Dry Suspension D-II | Dry Suspension E-III | Dry Suspension E-V |
|---|---|---|---|---|
| 0 | 0% | 0% | 0% | 0% |
| 10 | 35.5% | 38.7% | 34.5% | 15.6% |
| 15 | 58.4% | 59.6% | 46.8% | 20.5% |
| 20 | 79.5% | 83.5% | 59.4% | 29.5% |
| 30 | 86.5% | 96.5% | 78.5% | 36.4% |
| 45 | 92.5% | 95.4% | 91.2% | 45.9% |
| 60 | 93.2% | 94.6% | 92.5% | 60.5% |

In summary, the dry suspensions E-V made from the prescription in the ratio in the comparison, produces a suspension with comparable acid resistance to that of the present invention. However, its dissolution rate is significantly slowed down due to the highdosage of chitosan, resulting in the too high strength of gel, which affects the dissolution of the pellets.

It can be seen from the above results, in the suspension of eplazole according to the present invention, the combination of anionic gels and cationic polymers (especially chitosan and its derivatives) enables the dry suspension to rapidly reach a stable viscosity level and the prepared suspension gel can make the enteric pellets to suspend stably for a longer period of time. Furthermore, the dissolution rate of the acid-unstable proton pump inhibitors, especially Ilaprazole, can be increased.

## Claims

1. Dry suspension granules comprising anionic gels and cationic polymer, wherein the weight ratio of the anionic gels to the cationic polymer is (0.5∼50):1, preferably (0.8-20): 1, more preferably (0.9~10):1, and most preferably (1∼3):1.

2. The dry suspension granules of claim 1, wherein the anionic gels are selected from one, two or more of arabic gum, gelatin, alginate (e.g. sodium alginate), pectin, xanthan gum, gellan gum, locust bean gum, guar gum, agar, carrageenan, tamarind gum, konjac gum, cassia bean gum, tragacanth gum, and karaya gum.

3. The dry suspension granules of claim 1 or 2, wherein the cationic polymer is selected from chitosan or a derivative thereof; preferably, the weight percentage of the cationic polymer in dry suspension granules is 0.5-5%, preferably 0.6-4%, more preferably 0.8-3.5%, and most preferably 0.9-3.0%.

4. The dry suspension granules of any of claims 1-3, wherein the dry suspension granules according to the present invention further comprise one, two or more of adhesives, disintegrants, diluents, and pH regulators; preferably, the adhesives are selected from one, two or more of polyvinyl pyrrolidone, hydroxypropyl methyl cellulose, hydroxypropyl cellulose, methyl cellulose, polyvinyl alcohol, and starch; preferably, the disintegrants are selected from one, two or more of sodium carboxymethyl cellulose, calcium carboxymethyl cellulose, crosslinked sodium carboxymethyl cellulose, crosslinked sodium carboxymethyl starch, low-substituted hydroxypropyl cellulose, crosslinked polyvinyl pyrrolidone, microcrystalline cellulose, and pre-gelatinized starch; preferably, the diluents are selected from one, two or more of xylitol, mannitol, sucrose, glucose, sorbitol, maltitol, and fructose; preferably, the pH regulators are selected from organic or inorganic acids, preferably one, two or more of tartaric acid, citric acid, oxalic acid, succinic acid, fumaric acid, ascorbic acid, malic acid, glutamic acid, and caffeic acid.

5. The dry suspension granules of any of claims 1-4, wherein the pH value of suspension gel formed by the dry suspension granules in aqueous media ranges from 2.5 to 7.0, and preferably ranges from 3.0 to 5.0; and/or when preparing a suspension gel, the amount of a aqueous dispersion medium added into the dry suspension granules according to the present invention is 2 to 50 times of the weight of the dry suspension granules.

6. A method for preparing the dry suspension granules of any of claims 1-5, comprising the following steps:
(1) mixing anionic gels and cationic polymer, and optionally other excipients;
(2) preparing adhesive solutions separately;
(3) adding the adhesive solutions obtained in step (2) into the mixture obtained in step (1), to prepare a wetting mixture;
(4) granulating the wetting mixture obtained in step (3), to obtain the dry suspension granules according to the present invention;
preferably, the excipients comprise diluents, pH regulators, disintegrants, etc;
preferably, water and/or ethanol are used as solvent in step (2).

7. The method for preparing the dry suspension granules of any of claims 1-5, comprising the following steps:
(1) mixing anionic gels and cationic polymer, and optionally other excipients;
(2) providing water and/or ethanol as wetting agents;
(3) adding the wetting agents provided in step (2) into the mixture obtained in step (1), to prepare a wetting mixture;
(4) granulating the wetting mixture obtained in step (3), to obtain the dry suspension granules according to the present invention;
preferably, the other excipients comprise diluents, pH regulators, disintegrants, and/or adhesives, etc;
in the method for preparing dry suspension granules according to the present invention, preferably, further comprising a step (5) after step (4); step (5): drying the dry suspension granules according to the present invention;
in the method for preparing the dry suspension granules according to the present invention, preferably, further comprising a step (6) after step (5); step (6): granulating the dry suspension granules according to the present invention in the form of pellet.

8. A pharmaceutical composition, in particular a suspension, comprising the dry suspension granules of any of claims 1-5 or the dry suspension granules prepared with the method of claim 6 or claim 7; preferably, the pharmaceutical composition comprises a enteric pellet, in particular a enteric pellet comprising Ilaprazole; more preferably, the mass ratio of the dosage of Ilaprazole in the enteric pellets to the dosage of the dry suspension granules is 1:200~1000.

9. The pharmaceutical composition of claim 8, preferably a suspension, wherein after adding a dispersing medium to the pharmaceutical composition, the time taken for the obtained suspension gel to reach 75% of the maximum viscosity is less than 8 min, preferably less than 7 min; preferably, the time taken for the obtained suspension gel to reach 90% of the maximum viscosity is less than 12 min, preferably less than 11 min; preferably, the time taken for the formation of the suspension gel after the addition of an aqueous medium into the pharmaceutical composition is less than 120s, preferably less than 90s, more preferably less than 70s, and most preferably less than 50s; preferably, in the case of comprising enteric pellets that contain Ilaprazole, after adding the pharmaceutical composition into the dispersion medium and forming a solution with pH 1.2, the release rate of Ilaprazole not exceed 10% within 1h, and/or the release rate of Ilaprazole is not less than 70% within 45 min after forming a solution with pH 6.8.

10. A use of dry suspensions of any of claims 1-5 or the pharmaceutical compositions of claim 8 or claim 9 in the preparation of a medicament for treating and/or preventing gastrointestinal diseases; the gastrointestinal diseases comprise heartburn, inflammatory bowel disease, Crohn's disease, irritable bowel syndrome, ulcerative colitis, peptic ulcer, stress ulcer, bleeding peptic ulcer, duodenal ulcer and recurrent duodenal ulcer, gastric ulcer associated with NSAID, adult active benign gastric ulcer, infective enteritis, colitis, hyperacidity, dyspepsia, gastroparesis, Zollinger-Ellison syndrome, gastroesophageal reflux disease (GERD), diseases related to helicobacter pylori or eradication of helicobacter pylori, erosive esophagitis at all levels and short bowel syndrome, or any combination of the above diseases.
